# EUROPEAN PATENT SPECIFICATION

(11) **EP 2 906 182 B1**
(45) Date of publication and mention of the grant of the patent: **30.11.2016**
(21) Application number: 13773741.7
(22) Date of filing: 07.10.2013
(51) Int. Cl.: A61Q 11/00, A61K 8/02, A61K 8/19, A61K 8/25, A61K 8/27

(54) **ORAL CARE COMPOSITION**
MUNDPFLEGEMITTEL
COMPOSITION DE PROTECTION ORALE

(30) Priority: 12.10.2012 WO PCT/CN2012/082850; 16.11.2012 EP 12193011
(43) Date of publication of application: 19.08.2015
(73) Proprietor: Unilever N.V., 3013 AL Rotterdam (NL); Unilever PLC, London Greater London EC4Y 0DY (GB)
(72) Inventor: DENG, Yan, Shanghai, 200335 (CN); GUERY, Julie Savine Camille, Shanghai, 200335 (CN); LI, Xiaoke, Shanghai, 200335 (CN); LIU, Renjiang, Shanghai, 200335 (CN); ZHAO, Jing, Shanghai, 20050 (CN); ZHU, Yingjie, Shanghai, 20050 (CN)
(74) Representative: Tansley, Sally Elizabeth
(86) International application number: PCT/EP2013/070785
(87) International publication number: WO 2014/056824

(56) References cited:
- EP-A1- 0 539 651
- WO-A1-2008/006725
- WO-A2-00/00166
- GB-A- 1 535 031
- US-A- 4 048 300
- US-A- 5 468 489
- US-A1- 2009 010 857
- JIN WU ET AL: "Hierachically Nanostructured Mesoporous Spheres of Calcium Silicate Hydrate: Surfactant-Free Sonochemical Synthesis and Drug-Delivery System with Ultrahigh Drug-Loading Capacity", ADVANCED MATERIALS, WILEY VCH VERLAG, DE, vol. 22, 1 January 2010 (2010-01-01), pages 749-753, XP007921708, ISSN: 0935-9648, DOI: 10.1002/ADMA.200903020

## Description

### TECHNICAL FIELD OF THE INVENTION

The present invention relates to oral care compositions such as tooth pastes, gums, mouthwashes. In particular the present invention relates to oral care compositions containing a particle active comprising a calcium source and an antimicrobial metal cation. The invention also relates to such compositions for use in improving the oral hygiene of an individual and to processes for manufacturing the compositions.

### BACKGROUND TO THE INVENTION

Oral hygiene is a daily concern for most consumers. Unfortunately however, daily oral hygiene routines offer only limited protection against bacteria-mediated conditions and dental caries remains one of the most common diseases throughout the world.

To increase the antibacterial efficacy of oral care products, it is known to employ antibacterial metals such as zinc. Zinc can inhibit glycolysis and glucosyltransferases (GTFs) of bacterium even in a concentration as low as about 0.01 mM [Phan TN, Buckner T, Sheng J, et al., Oral Microbiology and Immunology, 2004, 19(1): 31-38]. Zinc has thus been formulated into oral-health products to give a range of benefits: It can reduce dental calculus formation, control plaque and reduce oral malodour.

Following dental hygiene practices such as tooth brushing, there is approximately 15-40% of total zinc in toothpaste that remains in the oral cavity but the zinc level in saliva and plaque falls rapidly over 30-60 minutes [Lynch, R. J. M., International Dental Journal. 61:46-54, 2011]. Thus efforts have been made to provide dentifrices having a longer lasting *in-situ* antibacterial benefit.

European patent application published as EP 0 539 651 A (SANGI CO, LTD) discloses a dentifrice having an antibacterial effect to prevent production of carious tooth and generation of periodontal diseases such as alveolar blennorrhea. The dentifrice contains hydroxylapatite powder. The hydroxylapatite powder carries therein an antibacterial metal such as silver, copper and/or zinc. The antibacterial metal is adsorbed to and/or combined, under ion exchange, with the hydroxylapatite powder. The antibacterial metal carried by the calcium compound is said to be highly stable such that the amount of released metal to water is extremely small, i.e., less than several ppb.

Unfortunately, by tightly combining the antibacterial metal with the hydroxyapatite, the metal ion may not necessarily be available to areas of the oral cavity (such as the soft tissue) away from the tooth surface and which may be reservoirs for caries-forming bacteria. In addition calcium phosphate salts such as hydroxyapatite may not deposit on teeth in a quantity sufficient to bind an antibacterial amount of metal to the tooth surface and/or do not convert to enamel-like layers on the tooth surface.

The present inventors have now recognized a need to provide an oral care product which is both efficient at depositing antibacterial metal on the tooth surface and which can release at least some of the antibacterial metal into saliva, and which in some embodiments at least may be capable of remineralizing teeth.

### TESTS AND DEFINITIONS

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purposes of the present invention means a paste or gel dentifrice for use with a toothbrush. Especially preferred are tooth pastes suitable for cleaning teeth by brushing for about two minutes.

### Mouth Wash

"Mouth wash" for the purposes of the present invention means liquid dentifrice for use in rinsing the mouth. Especially preferred are mouth washes suitable for rinsing the mouth by swishing and/or gargling for about half a minute before expectorating.

### In-Situ Solubility

*In-situ* solubility refers to dissolution of ions in oral fluid which, unless otherwise stated, is at a pH of 7.0. Such solubility can be determined *in-vitro* by measuring the amount of cation dissolved by immersing 1 part by weight particle active in 1000 parts by weight simulated oral fluid (SOF) at 37°C after shaking for 1 hour and whilst maintaining the pH at the desired value (within 0.1 pH units). For example, if the solubility at pH 7.0 is to be measured then the pH of the particle/SOF mixture is maintained at pH 7.0±0.1 for the whole 1 hour. The composition of SOF is given in Table 1 of the Examples herein below and Example 2 demonstrates the use of dropwise addition of HCl to maintain the pH of the SOF.

### Remineralization

"Remineralization", as used herein, means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### pH

When referring to the pH of an oral care composition, this means the pH measured when 1 part by weight of the composition is uniformly dispersed and/or dissolved in 20 parts by weight pure water at 25°C. In particular the pH may be measured by manually mixing 1 g oral care composition with 20 ml water for 30 s, then immediately testing the pH with indicator paper or a pH meter.

### Substantially Free

"Substantially free of", as used herein, means less than 1.5%, and preferably less than 1.0%, and more preferably less than 0.75% and more preferably still less than 0.5% and most preferably from 0.0 to 0.1 % by weight, based on total weight of the oral care composition, including all ranges subsumed therein.

### Viscosity

Viscosity of a tooth paste is the value taken at room temperature (25 °C) with a Brookfield Viscometer, Spindle No. 4 and at a speed of 5 rpm. Values are quoted in centipoises (cP = mPa.s) unless otherwise specified.

### Particle Size

"Particle size" as used herein means diameter size and reported as a weight average particle size. "Diameter" is meant to mean the longest length measurable in any dimension in the event the particle is not a perfect sphere. Particle size can be measured, for example by dynamic light scattering (DLS).

### Miscellaneous

All amounts are by weight of the final oral care composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis*

*mutandis.*

### SUMMARY OF THE INVENTION

In a first aspect, the present invention is directed to an oral care composition comprising:
a) particle active; and
b) physiologically acceptable carrier;
wherein the particle active comprises:
i) calcium source; and
ii) antimicrobial metal cation;
wherein the calcium source comprises calcium silicate, calcium carbonate or mixtures thereof,
wherein at least 0.05% by weight of total antimicrobial metal cation in the particle active is soluble *in situ*;
wherein the *in situ* solubility refers to dissolution of ions in oral fluid at a pH of 7.0.

In a second aspect, the invention is directed to an oral composition of the first aspect for use in improving the oral hygiene of an individual comprising applying the composition to at least one surface of the teeth of the individual. The method may additionally or alternatively be for remineralization of the teeth of the individual.

In a third aspect, the present invention is directed to a process for manufacturing the oral care composition of the first aspect wherein the process comprises the steps of:
i. forming particles of the calcium source;
ii. contacting the antimicrobial metal cation with the particles in a reaction solvent to form a reaction mixture;
iii. recovering the particle active from the reaction mixture; and
iv. combining the particle active with the physiologically acceptable carrier.

In a fourth aspect, the present invention is also directed to a process for manufacturing the oral care composition of the first aspect wherein the process comprises the steps of:
I. combining a calcium salt, anion source, antimicrobial metal cation source, and reaction solvent to form a reaction mixture;
II. simultaneously reacting the calcium salt and antimicrobial metal cation source with the anion source to form the particle active comprising calcium source and antimicrobial metal cation in the reaction mixture;
III. recovering the particle active from the reaction mixture; and
IV. combining the particle active with the physiologically acceptable carrier.

Compared to the process of the third aspect which requires the formation of particles of the calcium source in the first step, the process of the fourth aspect is easier to operate given the fact that the particle active is formed in a simultaneous reaction.

The present invention also provides the oral care composition of the first aspect obtained and/or obtainable by any embodiment of the processes of the invention.

All other aspects of the present invention will more readily become apparent upon considering the detailed description and examples which follow.

### DETAILED DESCRIPTION

The only limitation with respect to the particle active for use in the present invention is that the same is suitable for oral use and comprises antimicrobial cation and a calcium source. Without wishing to be bound by theory, the present inventors believe that the presence of the calcium source allows for effective deposition of the particle active onto teeth. In addition, the calcium source preferably has the ability to react with phosphate ions to produce a calcium phosphate *in situ* and so may allow for remineralizing dental tissue, such as enamel and/or dentin even when in normal use as part of a dentifrice and without any special procedures or implements.

The calcium source employed in this invention comprises calcium silicate, calcium carbonate or mixture thereof. Illustrative examples of further types of calcium source that may be used in this invention (owing to their ability to react with phosphate ions to produce a calcium phosphate *in situ*) include, for example, calcium gluconate, calcium oxide, calcium lactate, calcium hydroxide, calcium sulfate, calcium carboxymethyl cellulose, calcium alginate, calcium salts of citric acid, bioactive glass or a mixture thereof. More preferred, owing to their low water solubility and hence ability to form particles with long-term stability, are calcium oxide, calcium hydroxide, calcium sulfate, calcium carboxymethyl cellulose, calcium alginate, bioactive glass, mixtures thereof. In an especially preferred embodiment the calcium source is calcium silicate as such materials show exceptional biocompatibility. In a more preferred embodiment, the calcium silicate used is CaSiO₃ whereby the same is made commercially available under the name Microcal ET by PQ.

In yet another preferred embodiment, the calcium source is insoluble calcium silicate, present as the composite material calcium oxide-silica (CaO-SiO₂), such as is described, for example, in International patent application published as WO 2008/015117 (Unilever).

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the calcium source is a material having pores with diameters from 1 nm to 50 nm. Mesoporous calcium silicate (MCS) is often preferred. The MCS which may be used in this invention can be made, for example, by combining a calcium salt, a silica precursor like silicate and a structure-directing agent to yield a solid suitable for calcinating. A more detailed description of the process that may be conducted to make a MCS suitable for use in this invention is described in the aforementioned International application, published as WO 2008/015117.

Also suitable and often preferred is that the calcium source comprises or is calcium silicate hydrate (CSH), especially calcium silicate hydrate having a molar ratio of H₂O to Ca of from 10:1 to 1:5, more preferably from about 5:1 to 1:2, and most preferably from about 3:1 to 1:1. Mesoporous calcium silicate hydrate is described, for example, in J. Wu et al, Adv. Mater., 2010, 22, pp. 749-753.

Bioactive glass which may be used as the further calcium source comprises calcium and optionally phosphate ions. Suitable bioactive glasses are described, for example in WO 2010/041073 (BIOFILM LTD), WO 2009158564 (NOVAMIN TECHNOLOGY INC), WO 99/13852 (UNIV MARYLAND), WO 2005/063185 (NOVAMIN TECHNOLOGY INC), WO 96/10985 (BIOXID OY) and/or WO 97/27148 (UNIV MARYLAND).

To provide for optimum binding of the particle active to tooth surface, it is preferred that the particle active comprises the calcium source in an amount of at least 50% by weight of the particle active, more preferably at least 60%, more preferably still at least 65%, even more preferably at least 70%, even more preferably still at least 75% and most preferably from 90 to 99%.

The antimicrobial metal cation for use in this invention is preferably selected from a cation of copper, silver, zinc or a mixture thereof. Most preferred is zinc owing to its low toxicity and good antimicrobial properties.

To maximize the degree of antimicrobial effect delivered it is preferred that the particle active comprises at least 1 % antimicrobial metal cation by weight of the particle active, more preferably at least 3%, more preferably still at least 6%, even more preferably at least 9% and most preferably at least 20%. Antimicrobial ions can, however, have a bitter taste and therefore it is preferred that the particles comprise less than 40% antimicrobial metal cation by weight of the particle active, more preferably less than 35%, more preferably still less than 30% and most preferably less than 25%.

The amount of antimicrobial ions can be determined by various method such as EDX (Energy Dispersive X-ray Spectroscopy) and ICP-OES (Inductively Coupled Plasma Optical Emission Spectroscopy) in which ICP-OES is a preferred method.

The present inventors have recognized that it is advantageous that the antimicrobial metal cation is not too tightly bound to the particle active, otherwise the antimicrobial effect may be limited to the region of the tooth surface. Thus at least 0.05% by total weight of the total amount of antimicrobial metal cation in the particle active is soluble *in situ.* Preferably at least 0.08% by total weight of the amount of antimicrobial metal cation in the particle active is soluble *in situ,* more preferably at least 0.1 % and most preferably at least 0.2%. In order that the antimicrobial effect is durable, it is preferred that the antimicrobial metal cation is not released too quickly. In particular it is preferred that less than 20% by total weight of the antimicrobial metal cation in the particle active is soluble *in situ,* more preferably less than 10%, more preferably still less than 5%, and most preferably less than 2%.

In certain embodiments the antimicrobial cation can be released in response to acid. This means that the antimicrobial effect is potentially at its maximum when the tooth is most under threat, e.g. from acidic foodstuffs or from acidogenic bacteria. Thus the *in-situ* solubility of the antimicrobial metal cation in the particle active at pH 4.0 is preferably at least twice the *in-situ* solubility of the antimicrobial metal cation in the particle active at pH 7.0, more preferably at least three times and could even be from four to ten times. In other words, if the *in-situ* solubility of the antimicrobial metal in the particle active at pH 4.0 in weight % of total antimicrobial metal cation in the particle active is denoted n and the *in-situ* solubility of the antimicrobial metal in the particle active at pH 7.0 in weight % of total antimicrobial metal cation in the particle active is denoted m, then the ratio (n / m) is at least 2, more preferably at least 5, more preferably still at least 10, and most preferably from 20 to 500.

Additionally or alternatively it is preferred that at least 20% by weight of total antimicrobial metal cation in the particle active is soluble *in situ* at pH 4.0, more preferably at least 30%, more preferably still at least 50%, and most preferably from 70 to 100% .

Typically, the oral care composition of the present invention comprises from 0.1 to 60% by weight of the particle active, more preferably from 0.2 to 50%. Even more preferably the composition comprises the particle active in an amount of at least 0.3% by weight, more preferably still at least 0.5% or even at least 1 %. In a most preferred embodiment the composition comprises the particle active in an amount of at least 5% by weight, and optimally in the range 10 to 40% by weight.

Where the oral care composition is a tooth paste or powder, higher amounts of the particle active are preferred. This is because tooth pastes are typically applied only in small volumes (e.g. around 2 ml) per consumer use. In addition tooth pastes are typically opaque and therefore allow for incorporation of high levels of particles without affecting the appearance expected by the consumer. Thus in one embodiment, the oral care composition is a tooth paste or powder and comprises the particle active in an amount of at least 2% by weight, more preferably at least 5%, more preferably still at least 7 % by weight and most preferably in the range 10 to 40% by weight.

Where the oral care composition is a mouth wash, lower amounts of the particle active are preferred. This is because mouth washes are typically used in larger volumes (e.g. around 20 ml) per consumer use than tooth pastes. In addition mouth washes are typically transparent and therefore incorporation of high levels of particles may negatively affect the appearance expected by the consumer. Thus in one embodiment, the dentifrice is a mouth wash and comprises the particle active in an amount of at least 0.2% by weight, more preferably at least 0.5% by weight and most preferably from 1 to 20% by weight.

Preferably the particle active has a weight average particle size of five (5) microns or less, and more preferably from 10 to 100%, and especially, from 25 to 100%, and most especially, from 70 to 100% by weight of the particle active used in this invention has a particle size from 0.1 to 3 microns.

The particle active of the present invention may be capable of adhering to tooth surfaces even without inclusion of a phosphate source in the oral care composition itself. Without wishing to be bound by theory the present inventors believe that this may be because the calcium in the particle active reacts with phosphate ions in saliva and/or at the tooth surface.

Thus in one embodiment the oral care composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition). Presence of both the particle active and phosphate sources in a monophase hydrous formulation can lead to premature reaction of the calcium and phosphate and instability of the product.

For certain compositions, especially anhydrous compositions (i.e. compositions substantially free from water) or dual phase hydrous compositions, it is preferable to compound a phosphate source in the oral care composition to aid *in situ* generation of calcium phosphate. Thus the oral care composition preferably comprises phosphate source.

The phosphate source that may be used in this invention is limited only to the extent that the same may be used in a composition suitable for use in an oral cavity. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof. The phosphate source is preferably one which is water soluble, i.e., dissolves in water to give a solution with a concentration of at least 0.1 moles per litre.

Typically, the phosphate source makes up from 0.5 to 20%, and more preferably from 2 to 15%, and most preferably from 4 to 9% by weight of the oral care composition, based on total weight of the composition and including all ranges subsumed therein. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The phosphate source is preferably substantially free of calcium in order that the phosphate is able to react with the particle active to form calcium phosphate *in situ.* In certain embodiments the composition is substantially free of calcium phosphate salts (such as, for example, hydroxyapatite).

The oral care composition preferably has a pH of greater than 5.0. If the pH of the composition is too low then it may lower the pH in the oral cavity such that generation of *in situ* calcium phosphate is retarded and/or the antimicrobial metal cation may prematurely leak from the particle active. Therefore it is preferred that the pH of the oral care composition is in the range 6.0 to 11.0, more preferably 7.0 to 10.5 and most preferably 8.0 to 10.0.

The composition of the present invention comprises physiologically acceptable carrier. Typically the carrier comprises at least surfactant, thickener, humectant or a combination thereof.

Preferably the composition comprises a surfactant. Preferably the composition comprises at least 0.01 % surfactant by weight of the oral care composition, more preferably at least 0.1 % and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, and preferably, from 0.1 to 8%, and most preferably, from 1.5 to 6% by weight of the oral care composition, based on total weight of the composition and including all ranges subsumed therein.

When the dentifrice composition of this invention is a toothpaste, the same typically has a viscosity from about 50,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. Preferably, the humectant makes up from 25 to 80%, and most preferably, from 45 to 70% by weight of the oral care composition, based on total weight of the composition and including all ranges subsumed therein.

Oral care compositions described herein may comprise optional ingredients which are common in the art. These ingredients include antimicrobial agents, anti-inflammatory agents, anti-caries agents, plaque buffers, vitamins, fluoride sources, plant extracts, desensitizing agents, anti-calculus agents, biomolecules, flavors, proteinaceous materials, preservatives, opacifying agents (especially titanium dioxide), coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the oral care composition described herein, and preferably, from 0.0 to 15% by weight, and most preferably, from 0.01 to 12% by weight of the composition, including all ranges subsumed therein.

The oral care composition can be used in a method of improving the oral hygiene of an individual comprising applying the composition to at least one surface of the teeth of the individual. Additionally or alternatively the oral care composition may be used in a method of remineralizing the teeth of an individual comprising applying the composition to at least one surface of the teeth of the individual. The oral care composition of this invention may additionally or alternatively be used in the manufacture of a medicament for providing an oral care benefit as described herein, such as for example, for the treatment or prevention of dental caries.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 1 minute to 5 minutes. The composition may be used daily, for example for use by an individual once, twice or three times per day.

The composition may be manufactured by any convenient process provided that it yields a composition wherein at least 0.05% by total weight of antimicrobial metal cation in the particle active is soluble *in situ.* However, in a preferred embodiment the composition is manufactured by a process comprising the steps of:
i. forming particles of the calcium source;
ii. contacting the antimicrobial metal cation with the particles in a reaction solvent to form a reaction mixture;
iii. recovering the particle active from the reaction mixture; and
iv. combining the particle active with the physiologically acceptable carrier.

The step (i) of forming particles of the calcium source may comprise, for example, forming the particle active by reacting calcium salt with source of anion which forms an insoluble or slightly soluble salt with calcium. In this context "soluble", "insoluble" and "slightly soluble" refers to the solubility of a source (e.g., like calcium salts) in water at 25 °C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

The calcium salt may be a soluble calcium salt which is typically used in preparing porous (more preferably mesoporous) particles. Examples of soluble calcium salts include calcium chloride, calcium nitrate and mixtures thereof. Preferably, the calcium salt comprises or is insoluble calcium salts, slightly soluble calcium salts or a mixture thereof. Examples of slightly soluble or insoluble calcium salts include but not limited to calcium hydroxide, calcium oxide, calcium acetate, calcium lactate, calcium gluconate, calcium carbonate, calcium sulphate, calcium carboxymethyl cellulose, calcium alginate, calcium salts of citric acid or a mixture thereof. Examples of an anion which forms an insoluble or slightly soluble salt with calcium include oxide, silicate, carbonate, hydroxide, sulfate, carboxymethylcellulose, alginate and mixtures thereof. Where the anion is (or comprises) silicate the source of the anion may be, for example, alkyl orthosilicate such as TEOS (tetraethyl orthosilicate; Si(OCH₂CH₃)₄). An especially preferred method of forming particles of mesoporous calcium silicate hydrate is by reaction of such an alkyl orthosilicate with a soluble calcium salt whilst irradiating with sonic energy. Such a process is described, for example, in J. Wu et al, Adv. Mater., 2010, 22, pp. 749-753.

The step (ii) of contacting the antimicrobial metal ion with the particles to form a reaction mixture should be effected in such a way that the antimicrobial metal cation is not too tightly bound to the particle active. For example, the antimicrobial metal ion in the form of a solubilised salt could be infused into the particle. In a preferred embodiment, pre-formed particles of the calcium source are added to a solution of the antimicrobial metal cation in the reaction solvent to form the reaction mixture. In another preferred embodiment, the particles of calcium source are first formed in the reaction solvent and the antimicrobial metal cation is then added to form the reaction mixture. This latter embodiment is advantageous as it is a one-pot method.

The antimicrobial metal cation and the particles could be contacted in any suitable reaction solvent including water and other aqueous solvents. However, in some instances it may be suitable to provide the antimicrobial metal ion in the form of a salt and contact the salt with the particles in a reaction solvent wherein the solubility of the salt in the reaction solvent is lower than the solubility of the salt in water (under identical conditions of temperature and pressure). In such a manner the antimicrobial metal can be loaded into the particles in large amounts but still achieve the required solubility *in situ.*

The step (iii) typically comprises a solid-liquid separation step. For example, the particles may be separated from the reaction mixture by a process selected from centrifugation, sedimentation, filtration, drying or a combination thereof.

The step (iv) typically involves conventional formulation and mixing of ingredients to prepare an oral care formulation wherein such processes are well known to those skilled in the art.

In another preferred embodiment, the composition is manufactured by a process comprising the steps of:
I. combining a calcium salt, anion source, antimicrobial metal cation source, and reaction solvent to form a reaction mixture;
II. simultaneously reacting the calcium salt and antimicrobial metal cation source with the anion source to form the particle active comprising calcium source and antimicrobial metal cation in the reaction mixture;
III. recovering the particle active from the reaction mixture; and
IV. combining the particle active with the physiologically acceptable carrier.

Preferably, the calcium salt of the step (I) comprises or is insoluble calcium salts, slightly soluble calcium salts or a mixture thereof. Examples of slightly soluble or insoluble calcium salts include but not limited to calcium hydroxide, calcium oxide, calcium acetate, calcium lactate, calcium gluconate, calcium carbonate, calcium sulphate, calcium carboxymethyl cellulose, calcium alginate, calcium salts of citric acid or a mixture thereof. Examples of an anion source is one which forms an insoluble or slightly soluble salt with calcium include, for example oxide, silicate, carbonate, hydroxide, sulfate, carboxymethylcellulose, alginate and a mixture thereof. The antimicrobial metal ion source is preferably a salt of the antimicrobial metal, more preferably a soluble salt of the antimicrobial metal.

The formation of the particle active comprising calcium source and antimicrobial metal cation in a one pot reaction in step (II) may facilitate the incorporation of large amounts of antimicrobial metal cation into the particles.

The step (III) typically comprises a solid-liquid separation step. For example, the particles may be separated from the reaction mixture by a process selected from centrifugation, sedimentation, filtration, drying or a combination thereof.

The step (IV) typically involves conventional formulation and mixing of ingredients to prepare an oral care formulation wherein such processes are well known to those skilled in the art.

The following examples are provided to facilitate an understanding of the present invention. The examples are not provided to limit the scope of the claims.

### EXAMPLES

### Materials

Calcium nitrate (Ca(NO₃)₂·4H₂O), calcium hydroxide (Ca(OH)₂), TEOS, sodium hydroxide (NaOH), sodium metasilicate nonahydrate (Na₂SiO₃·9H₂O), zinc acetate (Zn(Ac)₂·2H₂O), zinc chloride (ZnCl₂), glycerol, ethanol and hydrochloric acid were purchased from Sinopharm Chemical Reagent Co., Ltd. Precipitated calcium carbonate (CaCO₃) was purchased from Maruo Calcium Co., Ltd. All the chemicals were used as received without further purification. 6 mm × 6 mm bovine enamel blocks, 6 cm-diameter glass dish and soft toothbrush were used for tooth brushing test. The SOF was prepared with ion concentrations and pH listed in Table 1.

**TABLE 1**

| | **NaCl** | **NaHCO₃** | **KCl** | **K₂HPO₄ ·3H₂O** | **MgCl₂· 6H₂O** | **HCl (1M)** | **CaCl₂** | **NaSO₄** | **Tris** |
|---|---|---|---|---|---|---|---|---|---|
| Conc. (mmol/L) | 137.35 | 4.165 | 3.005 | 7.17 | 0.153 | 20 | 0.90 | 0.505 | pH to 7.00 |

### Example 1

This example demonstrates the manufacture of particle active for use in the present invention.

### Two-Step Preparation

### Zn-MCSH

CSH mesoporous (MCSH) nanospheres were synthesized in a similar manner as described in J. Wu et al, Adv. Mater., 2010, 22, pp. 749-753. Briefly, 3.54 g Ca(NO₃)₂·4H₂O was dissolved into 500 mL deionized water. Then, 4 mL 5M NaOH aqueous solution and 2 mL TEOS were separately injected into the calcium nitrate solution. The resulting solution was stirred and ultrasonically irradiated for 20 minutes. The product was centrifuged, washed and collected for loading zinc ions.

The loading process was carried out in ethanol. Ethanol was chosen as solvent for zinc loading because ethanol is a poor solvent for zinc salts. Based on the experiments in our lab, the most dissolved amount of Zn(Ac)₂ in ethanol was 9.88 g/L. On the contrary, the solubility of Zn(Ac)₂ in water was 300 g/L.

As prepared MCSH wet powder was added into a flask with 500 mL ethanol where the concentration of Zn(Ac)₂·2H₂O was 2, 10, 20, 40 or 45 mmol/L, respectively. The above solution was stirred with a magnetic stirrer for 1 hour at room temperature (25 °C). The product was centrifuged, flushed by ethanol and dried at 60 °C in vacuum. The resultant powders were designated as Sample 1, Sample 2, Sample 3, Sample 4 and Sample 5. The composition of these various samples, as determined by elemental analysis by EDX (Energy Dispersive X-ray Spectroscopy), is given in Table 2. The composition of these samples was also analyzed using ICP-OES (Inductively Coupled Plasma Optical Emission Spectrometry). The samples were prepared as follows: 0.025g Zn/MCSH was added to 10mL 10% HNO₃ solution, shaken for 5 minutes and kept still overnight. The results are reported in Table 3 (error represents 95% confidence interval for duplicate measurements).

**TABLE 2**

| Sample | Concentration of Zn(Ac)₂ in manufacture (mmol/L) | Zn content in particles (wt. %) | Ca content in particles (wt. %) |
|---|---|---|---|
| **1** | 2 | 3.17 | 11.22 |
| **2** | 10 | 9.48 | 9.63 |
| **3** | 20 | 14.36 | 7.05 |
| **4** | 40 | 22.36 | 4.52 |
| **5** | 45 | 24.12 | 3.64 |

**TABLE 3**

| Sample | Concentration of Zn(Ac)₂ in manufacture (mmol/L) | Zn content in particles (wt. %) | Ca content in particles (wt. %) |
|---|---|---|---|
| **1** | 2 | 4.21 ±1.12 | 21.05 ± 0.16 |
| **2** | 10 | 13.56 ± 1.40 | 15.94 ± 0.57 |
| **3** | 20 | 19.43 ± 0.43 | 13.11 ± 0.21 |
| **4** | 40 | 19.81 ± 0.47 | 12.64 ± 0.09 |
| **5** | 45 | 20.12 ± 0.41 | 10.06 ± 0.16 |

The structural features of Zn/ MCSH particles were examined by electron microscopy. TEM (Transmission Electron Microscopy) and SEM (Scanning Electron Microscopy) micrographs revealed that the samples consisted of uniform isolated porous Zn/MCSH particles constructed by nanosheets. The diameters of hierarchical nanostructures ranged from 1 to 3 µm. All five samples had similar morphologies and sizes. This result confirmed that zinc diffused into the MCSH particles and did not form discrete precipitates. Taking Sample 5 as an example, the nitrogen-adsorption-desorption isotherm of Zn/ MCSH particles showed that after loading with Zn²⁺, the average pore size of MCSH decreased from 10.8 nm to 4.9 nm. Furthermore, Zn/ MCSH showed a particular pore size distribution with a sharp peak noted at around 4 nm where MCSH unloading showed a wide pore size distribution. The specific surface areas (SSA) of MCSH and Zn/ MCSH composite were also measured. The BET surface of MCSH before loading was 280 m²/g, after loading Zn in ethanol the specific surface area was measured as 261 m²/g.

### Zn/CSH or Zn/PCC

The CSH was prepared using a mixture of calcium hydroxide and sodium silicate in deionized water. The mixture was formed with an intial Ca:Si ratio of 1:1. The mixture was continuously stirred at room temperature (25°C). The pH of the reaction mixture was adjusted to and maintained around 11 using hydrochloric acid. After stirring for 5 hours the reaction mixture was filtered and the cake washed three times with water before again being filtered. The filter cake was dried in an oven at 80°C for 12 hours to yield the final CSH powder.

2.5g CSH or precipitated calcium carbonate (PCC) was added into 50mL 0.5mol/L ZnCl₂ solution and the resulted mixture was shaken for 1 hour then centrifuged to remove the supernatant. 30mL deionized water was added and the resulted solution was shaken for 1 minute, centrifuged to discard the supernatant. The water washing step was repeated for another two times. 20mL ethanol was added and the resulted solution was shaken for 1 minute, centrifuged to remove the supernatant and then the resultant powder was dried at 75°C for 6 hours. The dried powder was ground and sorted by 200 mesh sieve.

The resultant powders of Zn/PCC and Zn/CSH were designated as Sample 6 and Sample 7, respectively. The compositions of these samples were analyzed using ICP-OES, and the results are reported in Table 4 (error represents 95% confidence interval for duplicate measurements).

**TABLE 4**

| Sample | Zn content in particles (wt.%) | Ca content in particles (wt.%) |
|---|---|---|
| **6** | 22.9 ± 4.8 | 19.3 ± 4.7 |
| **7** | 19.0 ± 2.9 | 11.7 ± 0.60 |

The results confirmed that zinc was successfully loaded into PCC or CSH. The weight ratio of zinc to calcium was approximately 1.2:1 in Zn/PCC and 1.6:1 in Zn/CSH.

### One-Pot Preparation of Zn/CSH

### Loading Method

5.55g Ca(OH)₂ was dispersed in 150mL deionized water and the pH of the solution was adjusted to around 11.0 using 6mol/L or 0.5mol/L hydrochloric acid. 150mL 0.5mol/L Na₂SiO₃ solution was added slowly under continuous stirring. The pH of the resulted mixture was monitored throughout the experiment and any significant change from pH 11.0 was corrected by adding 6mol/L or 0.5mol/L hydrochloric acid dropwise. The reaction mixture was stirred for 5 hours, followed by addition of 10.2g ZnCl₂ then stirred for another 0.5 hour. The final pH of the reaction mixture was around 6.3. The resultant solid was filtered, washed with deionized water and ethanol, and dried at 105°C for 6 hours. The dried solid was ground and sorted by 200 mesh sieve.

### Co-precipitation Method

5.55g Ca(OH)₂ was dispersed in 150mL deionized water and pH of the solution was adjusted to around 11.0 using 6mol/L or 0.5mol/L hydrochloric acid. 75mL 1.0mol/L Na₂SiO₃ solution and 75mL 1.0mol/L ZnCl₂ solution were added slowly under continuous stirring. The pH of the resulting mixture was monitored throughout the experiment and any significant change from pH 11.0 was corrected by adding 6mol/L or 0.5mol/L hydrochloric acid dropwise. The reaction mixture was stirred for 6 hours. The resultant solid was filtered, washed with deionized water and ethanol, and dried at 105°C for 6 hours. The dried solid was ground and sorted by 200 mesh sieve.

The resultant powders of Zn/CSH prepared by loading method and co-precipitation method were designated as Sample 8 and Sample 9 respectively. The compositions of these samples were analyzed using ICP-OES, and the results are reported in Table 5 (error represents 95% confidence interval for duplicate measurements).

**TABLE 5**

| Sample | Zn content in particles (wt.%) | Ca content in particles (wt.%) |
|---|---|---|
| **8** | 37.8 ± 1.08 | 1.82 ± 0.03 |
| **9** | 32.4 ± 0.88 | 12.5 ± 0.98 |

The results demonstrated that zinc was successfully loaded into CSH, and it was easier for zinc to replace the calcium sites in CSH through loading method compared to co-precipitation method.

### Example 2

This example demonstrates the release of antimicrobial metal ions from particle active.

The particles used were the Zn-doped MCSH, PCC and CSH ones prepared as in Example 1.

0.05 g Zn/MCSH was immersed into 50 mL SOF where pH was 7.0 at 37 °C with continuous stirring. The sample containers were immersed in a water bath set at 37 °C throughout the experiment. The pH was monitored throughout the experiment and any significant change from pH 7 was corrected by drop-wise addition of 0.5 M HCl. After 60 minutes, the samples were centrifuged and the zinc content of the supernatant analyzed using ICP-OES. The results are shown in Table 6 (error represents 95% confidence interval for duplicate measurements).

**TABLE 6**

| Sample | Zn Concentration in Supernatant (mg / L) | Concentration of Zn in Supernatant (% of total Zn in particles) |
|---|---|---|
| **1** | 0.33 ± 0.09 | 1.0 ± 0.3 |
| **2** | 0.51 ± 0.03 | 0.53 ± 0.03 |
| **3** | 0.4 ± 0.2 | 0.3 ± 0.2 |
| **4** | 1.6 ± 0.6 | 0.7 ± 0.2 |
| **5** | 1.3 ± 0.2 | 0.5 ± 0.1 |

As can be seen from the data in Table 6, all of the Zn/MCSH particles released zinc ions into the SOF and had at least 0.3% *in situ* solubility of Zinc.

This experiment was repeated using Sample 3, except that SOF titrated to various pH values was used (0.5 M HCl was again used to adjust and maintain pH). The results are shown in Table 7 (error represents 95% confidence interval for duplicate measurements).

**TABLE 7**

| pH of SOF | Concentration of Zn in Supernatant (mg / L) |
|---|---|
| 7.0 | 4 ± 4 |
| 6.0 | 11.80 ± 0.02 |
| 5.5 | 56 ± 5 |
| 4.0 | 158 ± 3 |
| 2.0 | 167 ± 4 |

From this data we find that the more acidic the solution was, the more zinc ions were released. At pH 4 and below the Zn/MCSH dissolved and released substantially all of the zinc ions. This pH-activated release effect of zinc is promising for a responsive zinc release in oral cavity when stimulated by acid from drinking or acidogenic bacteria. Similar experiments were carried out for Samples 6 to 9, except a buffer solution with a pH of 5.50 was also used in addition to SOF with a pH of 7.0. 0.05g Zn/PCC or Zn/CSH was added to 20mL 1 mol/L acetate buffer solution of pH 5.50. The resulted solution was shaken for 16 hours at room temperature before centrifugation. The supernatant was filtered and measured using ICP-OES. The results are shown in Table 8 (error represents 95% confidence interval for duplicate measurements).

**TABLE 8**

| Sample | Concentration of Zn in Supernatant (% of total Zn in particles) pH 7.0 | Concentration of Zn in Supernatant (% of total Zn in particles) pH 5.50 |
|---|---|---|
| **6** | 0.19 ± 0.01 | 85.6 ± 13.6 |
| **7** | 0.16 ± 0.02 | 73.5 ± 2.65 |
| **8** | 0.51 ± 0.10 | 46.4 ± 3.63 |
| **9** | 0.19 ± 0.01 | 49.2 ± 2.06 |

As it is shown in Table 8, all Zn/PCC and Zn/CSH particles released zinc ions into the SOF or the buffer solution. The *in situ* solubility of zinc was at least 0.16% when the pH of SOF was 7.0. For Samples 6 and 7, which were prepared through two-step method, around 70 to 90% Zn was released when the pH of the buffer solution was 5.50. For Samples 8 and 9, which were prepared through one-step method, around 50% Zn was released when the pH of the buffer solution was 5.50.

### Example 3

This example demonstrates the deposition of particle active on tooth surfaces.

A paste containing 15 wt% Zn/MCSH in glycerol and a phosphate buffer solution containing 1.9 wt% Na₃PO₄ and 1.6% NaH₂PO₄ were prepared for tooth brushing test. The Zn/MCSH particles were the Zn-doped MCSH ones prepared as Sample 4 in Example 1. The bovine enamel blocks were brushed for 1 min using a slurry prepared just prior to brushing with 3.0 g of the paste and 6.0 mL of the phosphate buffer solution and immersed in the slurry for another 1 min. The bovine enamel blocks were washed for 2 times with deionized water and characterized using SEM for investigation of deposition. From the SEM images many Zn/MCSH particles deposited onto the enamel surface were apparent.

The brushing cycle was repeated for 14 times (two times each day to mimic a typical oral hygiene regime) and the bovine enamel blocks were incubated in SOF at 37 °C for at least 2 hours at the intervals. The bovine enamel blocks after 14 brushings were characterized using SEM and EDX. It was found from the SEM images that a dense and continuous layer was formed on the surface of the bovine enamel blocks. The composition of this layer (determined by EDX) is shown in Table 9:

**TABLE 9**

| **Element** | **Weight percentage (%)** | **Atom percentage (%)** |
|---|---|---|
| O | 29.59 | 50.63 |
| Mg | 0.89 | 1.00 |
| Si | 1.14 | 1.11 |
| P | 19.23 | 16.99 |
| Ca | 36.66 | 25.04 |
| Zn | 12.49 | 5.23 |

The results in this table show that the layer contained a considerable amount of zinc.

For Samples 6 to 9, the experiments were carried out as follows: 0.2g Zn/PCC or Zn/CSH powders was mixed with deionized water and put into the machine brushing dish with pellicle-coated bovine enamel blocks. Brushing was started immediately after addition of 5.0mL 0.23mol/L K₂HPO₄ solution (pH 9.27). Brushing lasted for 1 min and the bovine enamel blocks were immersed in the slurry for another 1 min. The bovine enamel blocks were washed 2 times with deionized water, dried at room temperature and characterized using SEM and EDX. The SEM images show that many Zn-PCC or Zn-CSH particles were successfully deposited onto the enamel surface. From the spectra of EDX, it clearly shows that zinc was deposited on the enamel surface after the treatment.

### Example 4

An example anhydrous monophase toothpaste formulation according to the invention is given in Table 10.

**TABLE 10**

| **Component** | **% w/w** |
|---|---|
| Glycerine | To 100 |
| PEG 400 | 10.50 |
| Flavour | 1.20 |
| Trisodium Phosphate | 3.80 |
| Zn-Doped Calcium Silicate Hydrate* and/or Zn-Doped Calcium Carbonate* | 15.00 |
| Sodium Monofluorophosphate | 1.11 |
| Sweetener | 0.20 |
| Monosodium Dihydrogen Phosphate | 3.20 |
| PEG 3000 | 1.75 |
| Pigment | 0.05 |
| Abrasive Silica | 7.00 |
| Sodium Lauryl Sulphate | 2.00 |

| | |
|---|---|
| *Samples 1 to 9 from Example 1 | |

### Example 5

An example dual-phase toothpaste formulation according to the invention is given in Table 11, wherein the phases can be combined in a weight ratio of about 1:1 immediately prior to use.

**TABLE 11**

| **Dual-phase toothpaste** | | | |
|---|---|---|---|
| **Calcium Phase** | | **Phosphate Phase** | |
| **Component** | **Content (wt%)** | **Composition** | **Content (wt%)** |
| Chlorinated water | 35.89 | Chlorinated Water | 3.768 |
| Sweetener | 0.20 | PEG1500 | 2.000 |
| Potassium Nitrate | 0.50 | Sweetener | 0.270 |
| Sorbitol(70%) | 20.00 | NaH₂PO₄ | 6.410 |
| Benz. Alcohol | 0.50 | Na₃PO₄ | 7.640 |
| Zn-Doped Calcium Silicate Hydrate* and/or Zinc-Doped Calcium Carbonate* | 30.00 | Sorbitol(70%) | 55.000 |
| Abrasive silica | 4.00 | Silica Abrasive | 12.000 |
| SCMC | 0.30 | Thickening Silica | 3.500 |
| Sodium Monofluorophosphate | 1.11 | Sodium Lauryl Sulphate | 6.600 |
| Flavour | 0.90 | Flavour | 1.200 |
| Sodium Lauryl Sulphate | 6.60 | SCMC | 0.500 |
| | | Sodium Monofluorophosphate | 1.110 |
| | | Colour | 0.002 |

| | | | |
|---|---|---|---|
| *Samples 1 to 9 from Example 1 | | | |

## Claims

1. An oral care composition comprising:
a) particle active; and
b) physiologically acceptable carrier;
wherein the particle active comprises:
i) calcium source; and
ii) antimicrobial metal cation;
wherein the calcium source comprises calcium silicate, calcium carbonate or mixtures thereof;
wherein at least 0.05% by total weight of antimicrobial metal cation in the particle active is soluble *in situ*;
wherein the *in situ* solubility refers to dissolution of ions in oral fluid at a pH of 7.0.

2. The oral care composition as claimed in claim 1, wherein the antimicrobial metal cation is selected from a cation of copper, silver, zinc or mixtures thereof.

3. The oral care composition as claimed in claim 2, wherein the antimicrobial metal cation is zinc.

4. The oral care composition as claimed in any one of the preceding claims, wherein the particle active comprises the antimicrobial metal cation in an amount of from 1 to 30% by weight of the particle active.

5. The oral care composition as claimed in any one of the preceding claims, wherein at least 0.1% by weight of total antimicrobial metal cation in the particle active is soluble *in situ,* preferably from 0.2 to 10%.

6. The oral care composition as claimed in any one of the preceding claims, wherein the *in-situ* solubility of the antimicrobial metal in the particle active at pH 4.0 is at least twice the *in-situ* solubility of the antimicrobial metal in the particle active at pH 7.0.

7. The oral care composition as claimed in any one of the preceding claims wherein at least 20% by weight of total antimicrobial metal cation in the particle active is soluble *in situ* at pH 4.0.

8. The oral care composition as claimed in any one of the preceding claims, wherein the calcium source comprises calcium silicate hydrate.

9. The oral care composition as claimed in any one of the preceding claims, wherein the calcium source is mesoporous.

10. The oral care composition as claimed in any preceding claim, wherein the composition is a dentifrice or chewing gum.

11. The oral care composition as claimed in claim 10, wherein the composition is a dentifrice, preferably a toothpaste.

12. The oral care composition as claimed in any one of the preceding claims, wherein the composition comprises a surfactant.

13. An oral care composition according to any of the preceding claims for use in improving the oral hygiene of an individual comprising applying the composition to at least one surface of the teeth of the individual.

14. A process for manufacturing the oral care composition as claimed in any one of claims 1 to 12 wherein the process comprises the steps of:
i. forming particles of the calcium source;
ii. contacting the antimicrobial metal cation with the particles in a reaction solvent to form a reaction mixture;
iii. recovering the particle active from the reaction mixture; and
iv. combining the particle active with the physiologically acceptable carrier.

15. The process as claimed in claim 14 wherein in step (ii) the antimicrobial metal ion is provided in the form of a salt and the salt is contacted with the particles in a reaction solvent wherein the solubility of the salt in the reaction solvent is lower than the solubility of the salt in water.

16. A process for manufacturing the oral care composition as claimed in any one of claims 1 to 12 wherein the process comprises the steps of:
I. combining a calcium salt, anion source, antimicrobial metal cation source, and reaction solvent to form a reaction mixture;
II. simultaneously reacting the calcium salt and antimicrobial metal cation source with the anion source to form the particle active comprising calcium source and antimicrobial metal cation in the reaction mixture;
III. recovering the particle active from the reaction mixture; and
IV. combining the particle active with the physiologically acceptable carrier.

## Patentansprüche

1. Mundpflegezusammensetzung, umfassend:
a) Partikelwirkstoff und
b) physiologisch verträglichen Träger,
wobei der Partikelwirkstoff umfasst:
i) Calciumquelle und
ii) antimikrobielles Metallkation,
wobei die Calciumquelle Calciumsilikat, Calciumcarbonat oder Mischungen davon umfasst,
wobei mindestens 0,05%, bezogen auf das Gesamtgewicht des antimikrobiellen Metallkations in dem Partikelwirkstoff, in situ löslich ist,
wobei sich die in situ-Löslichkeit auf das Auflösen von Ionen in einer Mundflüssigkeit bei einem pH von 7,0 bezieht.

2. Mundpflegezusammensetzung, wie im Anspruch 1 beansprucht, wobei das antimikrobielle Metallkation aus einem Kation von Kupfer, Silber, Zink oder Mischungen davon ausgewählt ist.

3. Mundpflegezusammensetzung, wie im Anspruch 2 beansprucht, wobei das antimikrobielle Metallkation Zink ist.

4. Mundpflegezusammensetzung, wie in irgendeinem der vorhergehenden Ansprüche beansprucht, wobei der Partikelwirkstoff antimikrobielles Metallkation in einer Menge von 1 bis 30 Gewichts-% des Partikelwirkstoffs umfasst.

5. Mundpflegezusammensetzung, wie in irgendeinem der vorhergehenden Ansprüche beansprucht, wobei mindestens 0,1 Gewichts-%, bezogen auf das Gesamtgewicht des antimikrobiellen Metallkations in dem Partikelwirkstoff, in situ löslich ist, vorzugsweise von 0,2 bis 10%.

6. Mundpflegezusammensetzung, wie in irgendeinem der vorhergehenden Ansprüche beansprucht, wobei die in situ-Löslichkeit des antimikrobiellen Metalls in dem Partikelwirkstoff bei pH 4,0 mindestens das Zweifache der in situ-Löslichkeit des antimikrobiellen Metalls in dem Partikelwirkstoff bei pH 7,0 beträgt.

7. Mundpflegezusammensetzung, wie in irgendeinem der vorhergehenden Ansprüche beansprucht, wobei mindestens 20%, bezogen auf das Gesamtgewicht des antimikrobiellen Metallkations in dem Partikelwirkstoff, bei pH 4,0 in situ löslich sind.

8. Mundpflegezusammensetzung, wie in irgendeinem der vorhergehenden Ansprüche beansprucht, wobei die Calciumquelle Calciumsilikathydrat umfasst.

9. Mundpflegezusammensetzung, wie in irgendeinem der vorhergehenden Ansprüche beansprucht, wobei die Calciumquelle mesoporös ist.

10. Mundpflegezusammensetzung, wie in irgendeinem vorhergehenden Anspruch beansprucht, wobei die Zusammensetzung ein Zahnputzmittel oder Kaugummi ist.

11. Mundpflegezusammensetzung, wie im Anspruch 10 beansprucht, wobei die Zusammensetzung ein Zahnputzmittel, vorzugsweise eine Zahnpaste, ist.

12. Mundpflegezusammensetzung, wie in irgendeinem der vorhergehenden Ansprüche beansprucht, wobei die Zusammensetzung ein Tensid umfasst.

13. Mundpflegezusammensetzung nach irgendeinem der vorhergehenden Ansprüche zur Verwendung bei der Verbesserung der Mundhygiene eines Individuums, umfassend das Auftragen der Zusammensetzung auf mindestens eine Oberfläche der Zähne des Individuums.

14. Verfahren zur Herstellung der Mundpflegezusammensetzung, wie in irgendeinem der Ansprüche 1 bis 12 beansprucht, wobei das Verfahren die Schritte umfasst:
i. Bilden von Partikeln der Calciumquelle,
ii. Inkontaktbringen des antimikrobiellen Metallkations mit den Partikeln in einem Reaktionslösungsmittel, um eine Reaktionsmischung zu bilden,
iii. Rückgewinnen des Partikelwirkstoffs aus der Reaktionsmischung und
iv. Kombinieren des Partikelwirkstoffs mit dem physiologisch verträglichen Träger.

15. Verfahren, wie im Anspruch 14 beansprucht, wobei in Schritt (ii) das antimikrobielle Metallion in Form eines Salzes geliefert und das Salz mit den Partikeln in einem Reaktionslösungsmittel in Kontakt gebracht wird,
wobei die Löslichkeit des Salzes in dem Reaktionslösungsmittel niedriger als die Löslichkeit des Salzes in Wasser ist.

16. Verfahren zur Herstellung der Mundpflegezusammensetzung, wie in irgendeinem der Ansprüche 1 bis 12 beansprucht, wobei das Verfahren die Schritte umfasst:
I. Kombinieren von Calciumsalz, Anionquelle, antimikrobieller Metallkation-Quelle und Reaktionslösungsmittel, um eine Reaktionsmischung zu bilden,
II. gleichzeitiges Umsetzen des Calciumsalzes und der antimikrobiellen Metallkation-Quelle mit der Anionquelle, um den Partikelwirkstoff zu bilden, umfassend Calciumquelle und antimikrobielles Metallkation in der Reaktionsmischung,
III. Wiedergewinnen des Partikelwirkstoffs aus der Reaktionsmischung und
IV. Kombinieren des Partikelwirkstoffs mit dem physiologisch verträglichen Träger.

## Revendications

1. Composition de soin oral comprenant :
a) un actif de particule ; et
b) un support physiologiquement acceptable ;
dans laquelle l'actif de particule comprend :
i) une source de calcium ; et
ii) un cation de métal antimicrobien ;
dans laquelle la source de calcium comprend du silicate de calcium, du carbonate de calcium ou des mélanges de ceux-ci ;
dans laquelle au moins 0,05 % en masse totale de cation de métal antimicrobien dans l'actif de particule est soluble *in-situ*;
dans laquelle la solubilité *in situ* fait référence à la dissolution d'ions dans un fluide oral à un pH de 7,0.

2. Composition de soin oral selon la revendication 1, dans laquelle le cation de métal antimicrobien est choisi parmi un cation de cuivre, d'argent, de zinc ou des mélanges de ceux-ci.

3. Composition de soin oral selon la revendication 2, dans laquelle le cation de métal antimicrobien est le zinc.

4. Composition de soin oral selon l'une quelconque des revendications précédentes, dans laquelle l'actif de particule comprend le cation de métal antimicrobien dans une quantité de 1 à 30 % en masse de l'actif de particule.

5. Composition de soin oral selon l'une quelconque des revendications précédentes, dans laquelle au moins 0,1 % en masse de cation de métal antimicrobien total dans l'actif de particule est soluble *in-situ,* de préférence de 0,2 à 10 %.

6. Composition de soin oral selon l'une quelconque des revendications précédentes, dans laquelle la solubilité *in-situ* du métal antimicrobien dans l'actif de particule à pH 4,0 est au moins deux fois la solubilité *in-situ* du métal antimicrobien dans l'actif de particule à pH 7,0.

7. Composition de soin oral selon l'une quelconque des revendications précédentes, dans laquelle au moins 20 % en masse de cation de métal antimicrobien total dans l'actif de particule sont solubles *in-situ* à pH 4,0.

8. Composition de soin oral selon l'une quelconque des revendications précédentes, dans laquelle la source de calcium comprend de l'hydrate de silicate de calcium.

9. Composition de soin oral selon l'une quelconque des revendications précédentes, dans laquelle la source de calcium est mésoporeuse.

10. Composition de soin oral selon l'une quelconque des revendications précédentes, dans laquelle la composition est un dentifrice ou un chewing-gum.

11. Composition de soin oral selon la revendication 10, dans laquelle la composition est un dentifrice, de préférence une pâte dentaire.

12. Composition de soin oral selon l'une quelconque des revendications précédentes, dans laquelle la composition comprend un tensioactif.

13. Composition de soin oral selon l'une quelconque des revendications précédentes pour une utilisation dans l'amélioration de l'hygiène orale d'un individu comprenant l'application de la composition sur au moins une surface des dents de l'individu.

14. Procédé de fabrication de la composition de soin oral selon l'une quelconque des revendications 1 à 12, dans lequel le procédé comprend les étapes de :
i. formation de particules de la source de calcium ;
ii. mise en contact du cation de métal antimicrobien avec les particules dans un solvant de réaction pour former un mélange réactionnel ;
iii. récupération de l'actif de particule du mélange réactionnel ; et
iv. combinaison de l'actif de particule avec le support physiologiquement acceptable.

15. Procédé selon la revendication 14, dans lequel dans l'étape (ii) l'ion de métal antimicrobien est fourni dans la forme d'un sel et le sel est mis en contact avec les particules dans un solvant de réaction dans lequel la solubilité du sel dans le solvant de réaction est inférieure à la solubilité du sel dans l'eau.

16. Procédé de fabrication de la composition de soin oral selon l'une quelconque des revendications 1 à 12, dans lequel le procédé comprend les étapes de :
I. combinaison d'un sel de calcium, d'une source d'anion, d'une source de cation de métal antimicrobien, et d'un solvant de réaction pour former un mélange réactionnel ;
II. réaction simultanée du sel de calcium et de la source de cation de métal antimicrobien avec la source d'anion pour former l'actif de particule comprenant une source de calcium et un cation de métal antimicrobien dans le mélange rédactionnel ;
III. récupération de l'actif de particule du mélange réactionnel ; et
IV. combinaison de l'actif de particule avec le support physiologiquement acceptable.
